# EUROPEAN PATENT APPLICATION

(11) **EP 1 375 644 A1**
(43) Date of publication of application: **02.01.2004**
(21) Application number: 03253988.4
(22) Date of filing: 25.06.2003
(51) Int. Cl.: C12N 1/16, C12N 13/00, A61K 41/00, A61K 35/72, A23L 1/28

(54) **Dietary supplements for treating hypertension**

(30) Priority: 28.06.2002 US 186504
(71) Applicant: Ultra Biotech Limited, Douglas IM1 1SA, Isle of Man (GB)
(72) Inventor: Cheung, Ling Yuk, New Territories Hong Kong (CN)
(74) Representative: Baldock, Sharon Claire

(57) **Abstract**

Compositions comprising a plurality of yeast cells, wherein said plurality of yeast cells are characterized by their ability to treat hypertension (e.g., lower blood pressure) in a subject (e.g., a mammal) as a result of having been cultured in the presence of an alternating electric field having a specific frequency and a specific field strength. Also included are methods of making and using such compositions.

## Description

### FIELD OF THE INVENTION

The invention relates to compositions that can ameliorate or prevent hypertension and are useful as dietary supplements (e.g., health drinks). These compositions contain yeast cells obtainable by growth in electromagnetic fields with specific frequencies and field strengths.

### BACKGROUND OF THE INVENTION

Hypertension is an insidious disease that affects many people. Hypertension is generally defined as an abnormally increased blood pressure. Hypertension is also a major indicator of the prognosis for future development of cardiovascular, cerebrovascular, and renal diseases. Certain risk factors, e.g., hypercholesterolemia, diabetes, smoking, and a familial history of vascular disease, in conjunction with hypertension, may predispose individuals to arteriosclerosis and consequent cardiovascular morbidity and mortality.

There are two major types of hypertension: essential (primary or idiopathic) and secondary hypertension. Hypertension of unknown etiology is termed essential hypertension, which is a relatively common disease state in people. Essential hypertension is generally asymptomatic and has been associated with the early onset of coronary disease, kidney failure and stroke. A combination of factors including age, obesity, smoking, heredity, stress, and an aggressive, hyperactive personality are thought to contribute to the occurrence of this form of hypertension.

Secondary hypertension is described as a symptom of a variety of underlying or primary diseases, such as renal vascular disease (e.g., atherosclerosis or stenosis of the renal artery) and renal parenchymal disease. Other disorders such as dysfunction of the adrenal cortex and medulla, atherosclerosis of the systemic arteries, and coarctation of the aorta may also produce secondary hypertension. The most common primary disorders leading to secondary hypertension are those that interfere with the supply of blood to the kidney. The resulting ischemia of renal tissue stimulates the secretion of renin, an enzyme that catalyzes the conversion of angiotensinogen to angiotensin I. Other enzymes in the body then convert angiotensin I to angiotensin II, the most potent vasoconstrictor known. Angiotensin II acts directly on the blood vessels and also acts as a physiologic stimulant to the adrenal gland and the production of aldosterone. Hence there is a twofold effect: (1) an increase in peripheral resistance due to vasoconstriction and a resulting elevation of blood pressure, and (2) a retention of sodium and water by the renal tubules in response to increased levels of serum aldosterone. The retained sodium and water increase blood volume, since they remain in the vascular system rather than being excreted by the kidney. The result is an increased cardiac output and an elevation of blood pressure.

Drug therapy is a common approach to treatment of hypertension. In general, antihypertensive drugs include diuretics, beta-blockers, angiotensin converting enzyme (ACE) inhibitors, anti-sympaticotonica, beta-blockers, calcium-antagonists and vasodilatators. However, each of these available drugs, though generally effective in reducing hypertension, has side effects, such as potassium depletion, hyperglycemia, hypokalemia, depression, carbohydrate intolerance, tachycardia, and/or allergic skin rashes, and in more severe cases, vomiting, fever, diarrhea, angina, and cardiac failure.

### SUMMARY OF THE INVENTION

This invention is based on the discovery that certain yeast cells can be activated by electromagnetic fields having specific frequencies and field strengths to produce substances useful in treating hypertension (e.g., both essential and secondary hypertension). Compositions comprising these activated yeast cells can therefore be used as dietary supplements, in the form of health drinks or dietary pills (tablets or powder). For instance, these compositions can be used to alleviate (e.g., lower) high blood pressure (systolic and/or diastolic pressure) in a hypertensive human, or to prevent or postpone the onset of hypertension in a high risk individual (e.g., someone predisposed to hypertension because of his genetic background or life style).

This invention embraces a composition comprising a plurality of yeast cells that have been cultured in an alternating electric field having a frequency in the range of about 11000 to 12000 MHz (e.g., 11500-11600 MHz) and a field strength in the range of about 180 to 500 mV/cm (e.g.,190-210, 210-250, 280-320, 320-350, 350-380, 380-420 or 420-450 mV/cm). The yeast cells are cultured for a period of time sufficient to activate said plurality of yeast cells to produce substances useful in treating hypertension in a subject. In one embodiment, the frequency and/or the field strength of the alternating electric field can be altered within the aforementioned ranges during said period of time. In other words, the yeast cells are exposed to a series of electromagnetic fields. An exemplary period of time is about 30-200 hours (e.g., 35-100 hours).

Also included in this invention is a composition comprising a plurality of yeast cells that have been cultured under acidic conditions in an alternating electric field having a frequency in the range of about 11000 to 12000 MHz (e.g., 11510-11550 MHz) and a field strength in the range of about 200 to 500 mV/cm (e.g., 380-420 mV/cm). In one embodiment, the yeast cells are exposed to a series of electromagnetic fields. An exemplary period of time is about 30-100 hours (e.g., 35-80 hours).

Yeast cells that can be included in this composition are available from the China General Microbiological Culture Collection Center ("CGMCC"), a depository recognized under the Budapest Treaty (China Committee for Culture Collection of Microorganisms, Institute of Microbiology, Chinese Academy of Sciences, Haidian, P.O. Box 2714, Beijing, 100080, China). Useful yeast species include, but are not limited to, those commonly used in food and pharmaceutical industries, such as *Saccharomyces cerevisiae, Saccharomyces* carlsbergensis, *Saccharomyces rouxii, Saccharomyces sake, Saccharomyces uvarum, Saccharomyces sp., Schizosaccharomyces pombe,* and *Rhodotorula aurantiaca.* For instance, the yeast cells can be of the strain *Saccharomyces carlsbergensis* Hansen AS2.420, AS2.440, or AS2.444; or *Saccharomyces cerevisiae* Hansen AS2.375, AS2.501, AS2.502, AS2.503, AS2.504, AS2.535, AS2.558, AS2.560, AS2.561, AS2.562, or IFFI1048. Other useful yeast strains are illustrated in Table 1.

This invention further embraces a composition comprising a plurality of yeast cells, wherein said plurality of yeast cells have been activated to treat hypertension in a subject. Included in this invention are also methods of making these compositions.

Unless otherwise defined, all technical and scientific terms used herein have the same meaning as commonly understood by one of ordinary skill in the art to which this invention belongs. Exemplary methods and materials are described below, although methods and materials similar or equivalent to those described herein can also be used in the practice or testing of the present invention. All publications and other references mentioned herein are incorporated by reference in their entirety. In case of conflict, the present specification, including definitions, will control. The materials, methods, and examples are illustrative only and not intended to be limiting. Throughout this specification and claims, the word "comprise," or variations such as "comprises" or "comprising" will be understood to imply the inclusion of a stated integer or group of integers but not the exclusion of any other integer or group of integers. A subject includes a human and veterinary subject.

Other features and advantages of the invention will be apparent from the following detailed description, and from the claims.

### BRIEF DESCRIPTION OF THE DRAWINGS

Fig. 1 is a schematic diagram showing an exemplary apparatus for activating yeast cells using electromagnetic fields. 1: yeast culture; 2: container; 3: power supply.
Fig. 2 is a schematic diagram showing an exemplary apparatus for making yeast compositions of the invention. The apparatus comprises a signal generator and interconnected containers 1, 2 and 3.

### DETAILED DESCRIPTION OF THE INVENTION

This invention is based on the discovery that certain yeast strains can be activated by electromagnetic fields ("EMF") having specific frequencies and field strengths to become highly efficient in producing substances that lower blood pressure (systolic and/or diastolic pressure) in a subject (e.g., a human subject). Compositions containing these activated yeast cells are therefore useful in the treatment of hypertension. Yeast compositions containing activated yeast cells can be used as dietary supplements, in the form of health drinks or dietary pills (tablets or powder).

Since the activated yeast cells contained in the yeast compositions have been cultured to endure acidic conditions (pH 2.5-4.2), these cells can survive the gastric environment and pass on to the intestines. Once in the intestines, the yeast cells are ruptured by various digestive enzymes, and the anti-hypertensive substances are released and readily absorbed.

Without being bound by any theory or mechanism, the inventor believes that EMFs activate or enhance the expression of a gene or a set of genes in yeast cells such that the yeast cells become active or more efficient in performing certain metabolic activities which lead to the desired anti-hypertensive effect.

### I. Yeast Strains Useful in the Invention

The types of yeasts useful in this invention include, but are not limited to, yeasts of the genera *Saccharomyces, Schizosaccharomyces pombe* and *Rhodotorula.*

Exemplary species within the above-listed genera include, but are not limited to, those illustrated in Table 1. Yeast strains useful for this invention can be obtained from laboratory cultures, or from publically accessible culture depositories, such as CGMCC and the American Type Culture Collection, 10801 University Boulevard, Manassas, VA 20110-2209. Non-limiting examples of useful strains (with accession numbers of CGMCC) are *Saccharomyces* *carlsbergensis* Hansen AS2.420, AS2.440, and AS2.444; *Saccharomyces cerevisiae* Hansen AS2.375, AS2.501, AS2.502, AS2.503, AS2.504, AS2.535, AS2.558, AS2.560, AS2.561, AS2.562, and IFFI1048. Other useful yeast strains are illustrated in Table 1.

Although it is preferred, the preparation of the yeast compositions of this invention is not limited to starting with a pure strain of yeast. A yeast composition of the invention may be produced by culturing a mixture of yeast cells of different species or strains. The ability of any activated species or strain of yeasts to treat hypertension can be readily tested by methods known in the art. See, for instance, Examples 1 and 2.

**Table 1**

| Exemplary Yeast Strains | | | | |
|---|---|---|---|---|
| *Saccharomyces cerevisiae* Hansen | | | | |
| ACCC2034 | ACCC2035 | ACCC2036 | ACCC2037 | ACCC2038 |
| ACCC2039 | ACCC2040 | ACCC2041 | ACCC2042 | AS2. 1 |
| AS2. 4 | AS2. 11 | AS2. 14 | AS2. 16 | AS2.56 |
| AS2. 69 | AS2. 70 | AS2. 93 | AS2. 98 | AS2. 101 |
| AS2. 109 | AS2. 110 | AS2. 112 | AS2. 139 | AS2. 173 |
| AS2. 174 | AS2. 182 | AS2. 196 | A52. 242 | A52. 336 |
| AS2. 346 | AS2. 369 | A52. 374 | AS2. 375 | AS2. 379 |
| A52. 380 | A52. 382 | AS2. 390 | AS2. 393 | A52. 395 |
| AS2.396 | AS2.397 | AS2.398 | AS2.399 | AS2.400 |
| AS2. 406 | AS2. 408 | AS2. 409 | AS2. 413 | AS2. 414 |
| AS2. 415 | AS2. 416 | AS2. 422 | AS2. 423 | AS2. 430 |
| AS2.431 | AS2.432 | AS2.451 | AS2.452 | AS2.453 |
| AS2. 458 | AS2. 460 | AS2. 463 | AS2. 467 | AS2. 486 |
| AS2.501 | AS2.502 | AS2. 503 | AS2. 504 | AS2.516 |
| AS2. 535 | AS2. 536 | AS2. 558 | AS2. 560 | AS2. 561 |
| AS2. 562 | AS2.576 | AS2. 593 | AS2. 594 | AS2.614 |
| AS2. 620 | AS2. 628 | AS2. 631 | AS2. 666 | AS2.982 |
| AS2. 1190 | AS2. 1364 | AS2. 1396 | IFFI1001 | IFFI1002 |
| IFFI1005 | IFFI1006 | IFFI1008 | IFFI1009 | IFFI1010 |
| IFFI1012 | IFFI1021 | IFFI1027 | IFFI1037 | IFFI1042 |
| IFFI1043 | IFFI1045 | IFFI1048 | IFFI1049 | IFFI1050 |
| IFFI1052 | IFFI1059 | IFFI1060 | IFFI1062 | IFFI1063 |
| IFFI1202 | IFFI1203 | IFFI1206 | IFFI1209 | IFFI1210 |
| IFFI1211 | IFFI1212 | IFFI1213 | IFFI1214 | IFFI1215 |
| IFFI1220 | IFFI1221 | IFFI1224 | IFFI1247 | IFFI1248 |
| IFFI1251 | IFFI1270 | IFFI1277 | IFFI1287 | IFFI1289 |
| IFFI1290 | IFFI1291 | IFFI1292 | IFFI1293 | IFFI1297 |
| IFFI1300 | IFFI1301 | IFFI1302 | IFFI1307 | IFFI1308 |
| IFFI1309 | IFFI1310 | IFFI1311 | IFFI1331 | IFFI1335 |
| IFFI1336 | IFFI1337 | IFFI1338 | IFFI1339 | IFFI1340 |
| IFFI1345 | IFFI1348 | IFFI1396 | IFFI1397 | IFFI1399 |
| IFFI1411 | IFFI1413 | IFFI1441 | IFFI1443 | |
| *Saccharomyces cerevisiae* Hansen Var. ellipsoideus (Hansen) Dekker | | | | |
| ACCC2043 | AS2.2 | AS2.3 | AS2.8 | AS2.53 |
| AS2.163 | AS2.168 | AS2.483 | AS2.541 | AS2.559 |
| AS2.606 | AS2.607 | AS2.611 | AS2.612 | |
| *Saccharomyces chevalieri* Guilliermond | | | | |
| AS2.131 | AS2.213 | | | |
| *Saccharomyces delbrueckii* | | | | |
| AS2.285 | | | | |
| *Saccharomyces delbrueckii* Lindner ver. mongolicus (Saito) Lodder et van Rij | | | | |
| AS2.209 | AS2.1157 | | | |
| *Saccharomyces exiguous* Hansen | | | | |
| AS2.349 | AS2.1158 | | | |
| *Saccharomyces fermentati* (Saito) Lodder et van Rij | | | | |
| AS2.286 | AS2.343 | | | |
| *Saccharomyces logos* van laer et Denamur ex Jorgensen | | | | |
| AS2.156 | AS2.327 | AS2.335 | | |
| *Saccharomyces mellis* (Fabian et Quinet) Lodder et kreger van Rij | | | | |
| AS2.195 | | | | |
| *Saccharomyces mellis* Microellipsoides Osterwalder | | | | |
| AS2.699 | | | | |
| *Saccharomyces oviformis* Osteralder | | | | |
| AS2.100 | | | | |
| *Saccharomyces rosei* (Guilliermond) Lodder et Kreger van Rij | | | | |
| AS2.287 | | | | |
| *Saccharomyces rouxii* Boutroux | | | | |
| AS2.178 | AS2.180 | AS2.370 | AS2.371 | |
| *Saccharomyces sake* Yabe | | | | |
| ACCC2045 | | | | |
| *Candida arborea* | | | | |
| AS2.566 | | | | |
| *Candida lambica* (Lindner et Genoud) van. Uden et Buckley | | | | |
| AS2.1182 | | | | |
| *Candida krusei* (Castellani) Berkhout | | | | |
| AS2.1045 | | | | |
| *Candida lipolytica* (Harrison) Diddens et Lodder | | | | |
| AS2.1207 | AS2.1216 | AS2.1220 | AS2.1379 | AS2.1398 |
| AS2.1399 | AS2.1400 | | | |
| *Candida parapsilosis* (Ashford) Langeron et Talice Var. intermedia Van Rij et Verona | | | | |
| AS2.491 | | | | |
| *Candida parapsilosis* (Ashford) Langeron et Talice | | | | |
| AS2.590 | | | | |
| *Candida pulcherrima* (Lindner) Windisch | | | | |
| AS2.492 | | | | |
| *Candida rugousa* (Anderson) Diddens et Lodder | | | | |
| AS2.511 | AS2.1367 | AS2.1369 | AS2.1372 | AS2.1373 |
| AS2.1377 | AS2.1378 | AS2.1384 | | |
| *Candida tropicalis* (Castellani) Berkhout | | | | |
| ACCC2004 | ACCC2005 | ACCC2006 | AS2.164 | AS2.402 |
| AS2.564 | AS2.565 | AS2.567 | AS2.568 | AS2.617 |
| AS2.637 | AS2.1387 | AS2.1397 | | |
| *Candida utilis* Henneberg Lodder et Kreger Van Rij | | | | |
| AS2.120 | AS2.281 | AS2.1180 | | |
| *Crebrothecium ashbyii* (Guillermond) Routein *(Eremothecium ashbyii* Guilliermond) | | | | |
| AS2.481 | AS2.482 | AS2.1197 | | |
| *Geotrichum candidum* Link | | | | |
| ACCC2016 | AS2.361 | AS2.498 | AS2.616 | AS2.1035 |
| AS2.1062 | AS2.1080 | AS2.1132 | AS2.1175 | AS2.1183 |
| *Hansenula anomala* (Hansen)H et P sydow | | | | |
| ACCC2018 | AS2.294 | AS2.295 | AS2.296 | AS2.297 |
| AS2.298 | AS2.299 | AS2.300 | AS2.302 | AS2.338 |
| AS2.339 | AS2.340 | AS2.341 | AS2.470 | AS2.592 |
| AS2.641 | AS2.642 | AS2.782 | AS2.635 | AS2.794 |
| *Hansenula arabitolgens* Fang | | | | |
| AS2.887 | | | | |
| *Hansenula jadinii* (A. et R Sartory Weill et Meyer) Wickerham | | | | |
| ACCC2019 | | | | |
| *Hansenula saturnus* (Klocker) H et P sydow | | | | |
| ACCC2020 | | | | |
| *Hansenula schneggii* (Weber ) Dekker | | | | |
| AS2.304 | | | | |
| *Hansenula subpelliculosa* Bedford | | | | |
| AS2.740 | AS2.760 | AS2.761 | AS2.770 | AS2.783 |
| AS2.790 | AS2.798 | AS2.866 | | |
| *Kloeckera apiculata* (Reess emend. Klocker) Janke | | | | |
| ACCC2022 | ACCC2023 | AS2.197 | AS2.496 | AS2.714 |
| ACCC2021 | AS2.711 | | | |
| *Lipomycess starkeyi* Lodder et van Rij | | | | |
| AS2.1390 | ACCC2024 | | | |
| *Pichia farinosa* (Lindner) Hansen | | | | |
| ACCC2025 | ACCC2026 | AS2.86 | AS2.87 | AS2.705 |
| AS2.803 | | | | |
| *Pichia membranaefaciens* Hansen | | | | |
| ACCC2027 | AS2.89 | AS2.661 | AS2.1039 | |

| *Rhodosporidium toruloides* Banno | | | | |
|---|---|---|---|---|
| ACCC2028 | | | | |
| *Rhodotorula glutinis* (Fresenius) Harrison | | | | |
| AS2.2029 | AS2.280 | ACCC2030 | AS2.102 | AS2.107 |
| AS2.278 | AS2.499 | AS2.694 | AS2.703 | AS2.704 |
| AS2.1146 | | | | |
| *Rhodotorula minuta* (Saito) Harrison | | | | |
| AS2.277 | | | | |
| *Rhodotorula rubar* (Demme) Lodder | | | | |
| AS2.21 | AS2.22 | AS2.103 | AS2.105 | AS2.108 |
| AS2.140 | AS2.166 | AS2.167 | AS2.272 | AS2.279 |
| AS2.282 | ACCC2031 | | | |
| *Rhodotorula aurantiaca* (Saito) Lodder | | | | |
| AS2.102 | AS2.107 | AS2.278 | AS2.499 | AS2.694 |
| AS2.703 | AS2.704 | AS2.1146 | | |

| *Saccharomyces carlsbergensis* Hansen | | | | |
|---|---|---|---|---|
| AS2.113 | ACCC2032 | ACCC2033 | AS2.312 | AS2.116 |
| AS2.118 | AS2.121 | AS2.132 | AS2.162 | AS2.189 |
| AS2.200 | AS2.216 | AS2.265 | AS2.377 | AS2.417 |
| AS2.420 | AS2.440 | AS2.441 | AS2.443 | AS2.444 |
| AS2.459 | AS2.595 | AS2.605 | AS2.638 | AS2.742 |
| AS2.745 | AS2.748 | AS2.1042 | | |
| *Saccharomyces uvarum* Beijer | | | | |
| IFFI1023 | IFFI1032 | IFFI1036 | IFFI1044 | IFFI1072 |
| IFFI1205 | IFFI1207 | | | |
| *Saccharomyces willianus* Saccardo | | | | |
| AS2.5 AS2.7 | AS2.119 | AS2.152 | AS2.293 | |
| AS2.381 | AS2.392 | AS2.434 | AS2.614 | AS2.1189 |
| *Saccharomyces sp.* | | | | |
| AS2.311 | | | | |
| *Saccharomycodes ludwigii* Hansen | | | | |
| ACCC2044 | AS2.243 | AS2.508 | | |
| *Saccharomycodes sinenses* Yue | | | | |
| AS2.1395 | | | | |
| *Schizosaccharomyces octosporus* Beijerinck | | | | |
| ACCC2046 | AS2.1148 | | | |
| *Schizosaccharomyces pombe* Lindner | | | | |
| ACCC2047 | ACCC2048 | AS2.214 | AS2.248 | AS2.249 |
| AS2.255 | AS2.257 | AS2.259 | AS2.260 | AS2.274 |
| AS2.994 | AS2.1043 | AS2.1149 | AS2.1178 | IFFI1056 |
| *Sporobolomyces roseus* Kluyver et van Niel | | | | |
| ACCC2049 | ACCC2050 | AS2.19 | AS2.962 | AS2.1036 |
| ACCC2051 | AS2.261 | AS2.262 | | |
| *Torulopsis candida* (Saito) Lodder | | | | |
| AS2.270 | ACCC2052 | | | |
| *Torulopsis famta* (Harrison) Lodder et van Rij | | | | |
| ACCC2053 | AS2.685 | | | |
| *Torulopsis globosa* (Olson et Hammer) Lodder et van Rij | | | | |
| ACCC2054 | AS2.202 | | | |
| *Torulopsis inconspicua* Lodder et Kreger van Rij | | | | |
| AS2.75 | | | | |
| *Trichosporon behrendii* Lodder et Kreger van Rij | | | | |
| ACCC2056 | AS2.1193 | | | |
| *Trichosporon capitatum* Diddens et Lodder | | | | |
| ACCC2056 | AS2.1385 | | | |
| *Trichosporon cutaneum* (de Beurm et al.) Ota | | | | |
| ACCC2057 | AS2.25 | AS2.570 | AS2.571 | AS2.1374 |
| *Wickerhamia fluorescens* (Soneda) Soneda | | | | |
| ACCC2058 | AS2.1388 | | | |

### II. Application of Electromagnetic Fields

An electromagnetic field useful in this invention can be generated and applied by various means well known in the art. For instance, the EMF can be generated by applying an alternating electric field or an oscillating magnetic field.

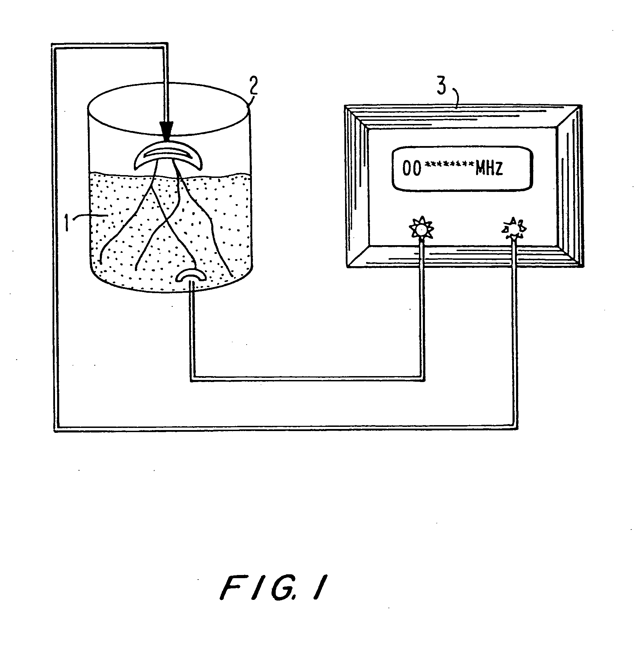

Alternating electric fields can be applied to cell cultures through electrodes in direct contact with the culture medium, or through electromagnetic induction. See, e.g., Fig. 1. Relatively high electric fields in the medium can be generated using a method in which the electrodes are in contact with the medium. Care must be taken to prevent electrolysis at the electrodes from introducing undesired ions into the culture and to prevent contact resistance, bubbles, or other features of electrolysis from dropping the field level below that intended. Electrodes should be matched to their environment, for example, using Ag-AgCl electrodes in solutions rich in chloride ions, and run at as low a voltage as possible. For general review, see Goodman et al., *Effects of EMF on Molecules and Cells,* International Review of Cytology, A Survey of Cell Biology, Vol. 158, Academic Press, 1995.

The EMFs useful in this invention can also be generated by applying an oscillating magnetic field. An oscillating magnetic field can be generated by oscillating electric currents going through Helmholtz coils. Such a magnetic field in turn induces an electric field.

The frequencies of EMFs useful in this invention range from about 11000 to 12000 MHz (e.g., 11500-11600 MHz). Exemplary frequencies are 11508,11515,11521, 11528, and 11535MHz. The field strength of the electric field useful in this invention ranges from about 180 to 500 mV/cm (e.g., 190-210, 210-250, 280-320, 320-350, 350-380, 380-420 or 420-450 mV/cm). Exemplary field strengths are 200, 223, 310, 316, 332, 364, 406, 413, 433, and 435 mV/cm.

When a series of EMFs are applied to a yeast culture, the yeast culture can remain in the same container while the same set of EMF generator and emitters is used to change the frequency and/or field strength. The EMFs in the series can each have a different frequency or a different field strength; or a different frequency and a different field strength. Such frequencies and field strengths are preferably within the above-described ranges. Although any practical number of EMFs can be used in a series, it may be preferred that the yeast culture be exposed to a total of 2, 3, 4, 5, 6, 7, 8, 9 or 10 EMFs in a series.

Although the yeast cells can be activated after even a few hours of culturing in the presence of an EMF, it may be preferred that the activated yeast cells be allowed to multiply and grow in the presence of the EMF(s) for a total of 30-200 hours (e.g., 35-100 hours).

Fig. 1 illustrates an exemplary apparatus for generating alternating electric fields. An electric field of a desired frequency and intensity is generated by an AC source (3) capable of generating an alternating electric field, preferably in a sinusoidal wave form, in the frequency range of 10 to 20,000 MHz. Signal generators capable of generating signals with a narrower frequency range can also be used. If desirable, a signal amplifier can also be used to increase the output. The activation container (2) can be made from non-conductive metal material, e.g., plastics, glass or ceramic. The wire connecting the activation container (2) and the signal generator (3) is preferably a high frequency coaxial cable with a transmission frequency of at least 30 GHz.

The alternating electric field can be applied to the culture by a variety of means, including placing the yeast culture (1) in close proximity to the signal emitters such as a metal wire or tube capable of transmitting EMFs. The metal wire or tube can be made of red copper, and be placed inside the container (2), reaching as deep as 3-30 cm. For example, if the fluid in the container (2) has a depth of 15-20 cm, 20-30 cm, 30-50 cm, 50-70 cm, 70-100 cm, 100-150 cm or 150-200 cm, the metal wire can be 3-5 cm, 5-7 cm, 7-10 cm, 10-15 cm, 15-20 cm, 20-30 cm and 25-30 cm from the bottom of the container (2), respectively. The number of electrode wires used depends on the volume of the culture as well as the diameter of the wires. The number of metal wires/tubes used can be from 1 to 10 (e.g., 2 to 3). It is recommended, though not mandated, that for a culture having a volume up to 10 L, metal wires/tubes having a diameter of 0.5 to 2.0 mm be used. For a culture having a volume between 10 L and 100 L, metal wires/tubes having a diameter of 3.0 to 5.0 mm can be used. For a culture having a volume in the range of 100-1000 L, metal wires/tubes having a diameter of 6.0 to 15.0 mm can be used. For a culture having a volume greater than 1000 L, metal wires/tubes having a diameter of 20.0 to 25.0 mm can be used.

In one embodiment, the electric field is applied by electrodes submerged in the culture (1). In this embodiment, one of the electrodes can be a metal plate placed on the bottom of the container (2), and the other electrode can comprise a plurality of electrode wires evenly distributed in the culture (1) so as to achieve even distribution of the electric field energy. The number of electrode wires used depends on the volume of the culture as well as the diameter of the wires.

### III. Culture Media

Culture media useful in this invention contain sources of nutrients assimilable by yeast cells. Complex carbon-containing substances in a suitable form, such as carbohydrates (e.g., sucrose, glucose, fructose, dextrose; maltose, xylose, cellulose, starches, etc.) and coal, can be the carbon sources for yeast cells. The exact quantity of the carbon sources utilized in the medium can be adjusted in accordance with the other ingredients of the medium. In general, the amount of carbohydrates varies between about 0.1% and 10% by weight of the medium and preferably between about 0.1% and 5% (e.g., about 2%). These carbon sources can be used individually or in combination. Amino acid-containing substances in suitable form (e.g., beef extract and peptone) can also be added individually or in combination. In general, the amount of amino acid containing substances varies between about 0.1 % and 0.5% by weight of the medium and preferably between about 0.1% and 0.3% (e.g., about 0.25%). Among the inorganic salts which can be added to the culture medium are the customary salts capable of yielding sodium, potassium, calcium, phosphate, sulfate, carbonate, and like ions. Non-limiting examples of nutrient inorganic salts are (NH₄)₂HPO₄, KH₂PO₄, K₂HPO₄, CaCO₃, MgSO₄, NaC1, and CaSO₄.

### IV. Electromagnetic Activation of Yeast Cells

To activate or enhance the ability of yeast cells to produce substances beneficial for the treatment of hypertension (e.g., lowering of blood pressure), these cells can be activated by being cultured in an appropriate medium under sterile conditions at 20°C-38°C, preferably at 28-32°C (e.g., 30°C) for a sufficient amount of time, e.g., 30-200 hours (eg., 35-100 hours), in an alternating electric field or a series of alternating electric fields as described above.

An exemplary culture medium is made by mixing 1000 ml of distilled water with 20 g of sucrose, 30 µg of vitamin B₃, 60 µg of vitamin H, 30 µg of vitamin B_{12,} 0.20 g of KH₂PO₄, 0.2 g of MgSO₄•7H₂O, 0.25 g of NaC1, 0.1 g of CaSO₄•2H₂O, 3.0 g of CaCO₃•5H₂O, and 2.5 g of peptone.

An exemplary set-up of the culturing process is depicted in Fig. 1. Untreated yeast cells are added to a culture medium at 1x10 ⁸ cells per 1000 ml of the culture medium. The yeast cells may be *Saccharomyces cerevisiae* Hansen AS2.561, or may be selected from any of the strains listed in Table 1. An exemplary activation process of the yeast cells involves the following sequence: the yeast cells are grown in the culture medium for 38-42 hours (e.g., 40 hours) at 28-32°C and then exposed to (1) an alternating electric field having a frequency of 11508 MHz and a field strength in the range of 280-320 mV/cm (e.g., 310 mV/cm) for 16-22 hours (e.g., 20 hours); (2) then to an alternating electric field having a frequency of 11515 MHz and a field strength in the range of 280-320 mV/cm (e.g., 316 mV/cm) for 16-22 hours (e.g., 20 hours); (3) then to an alternating electric field having a frequency of 11521 MHz and a field strength in the range of 350-380 mV/cm (e.g., 364 mV/cm) for 20-25 hours (e.g., 23 hours); (4) then to an alternating electric field having a frequency of 11528 MHz and a field strength in the range of 420-450 mV/cm (e.g., 435 mV/cm) for 16-22 hours (e.g., 21 hours); and (5) finally to an alternating electric field having a frequency of 11535 MHz and a field strength in the range of 420-450 mV/cm (e.g., 433 mV/cm) for 15-22 hours (e.g., 15 hours). The activated yeast cells are then recovered from the culture medium by various methods known in the art, dried (e.g., by lyophilization) and stored at about 4°C in powder form. The resultant yeast powder preferably contains no less than 10¹⁰ cells/g activated yeast.

Subsequently, the activated yeast cells can be evaluated for their ability to treat hypertension using standard methods known in the art, such as those described in Section VII.

### V. Acclimatization of Yeast Cells To the Gastric Environment

Because the activated yeast cells of this invention must pass through the stomach before reaching the small intestine, where the effective components are released from these yeast cells, it is preferred that these yeasts be cultured under acidic conditions so as to acclimatize the cells to the gastric juice. This acclimatization process results in better viability of the yeast cells in the acidic gastric environment.

To achieve this, the yeast powder containing activated yeast cells can be mixed with a highly acidic acclimatizing culture medium at 10 g (containing more than 10¹⁰ activated cells per gram) per 1000 ml. The yeast mixture can then be cultured first in the presence of an alternating electric field having a frequency of 11528 MHz and a field strength in the range of 380-420 mV/cm (e.g., 406 mV/cm) at about 28 to 32°C for 36- 42 hours (e.g., 38 hours). The resultant yeast cells can then be further incubated in the presence of an alternating electric field having a frequency of 11535 MHz and a field strength in the range of 380-420 mV/cm (e.g., 413 mV/cm) at about 28 to 32°C for 20-42 hours (e.g., 20 hours). The resulting acclimatized yeast cells are then recovered from the culture medium by various methods known in the art and are either dried and stored in powder form (≥10¹⁰ cells/g) at room temperature or in vacuum at 0-4°C.

An exemplary acclimatizing culture medium is made by mixing 700 ml fresh pig gastric juice and 300 ml wild Chinese hawthorn extract. The pH of acclimatizing culture medium is adjusted to 2.5 with 0.1 M hydrochloric acid (HC1) and 0.2 M potassium biphthalate (C₆H₄(COOK)COOH). The fresh pig gastric juice is prepared as follows. At about 4 months of age, newborn Holland white pigs are sacrificed, and the entire contents of their stomachs are retrieved and mixed with 2000 ml of water under sterile conditions. The mixture is then allowed to stand for 6 hours at 4°C under sterile conditions to precipitate food debris. The supernatant is collected for use in the acclimatizing culture medium. To prepare the wild Chinese hawthorn extract, 500 g of fresh wild Chinese hawthorn is dried under sterile conditions to reduce water content (≤8%). The dried fruit is then ground (≥20 mesh) and added to 1500 ml of sterile water. The hawthorn slurry is allowed to stand for 6 hours at 4°C under sterile conditions. The hawthorn supernatant is collected to be used in the acclimatizing culture medium.

### VI. Manufacture of Yeast Compositions

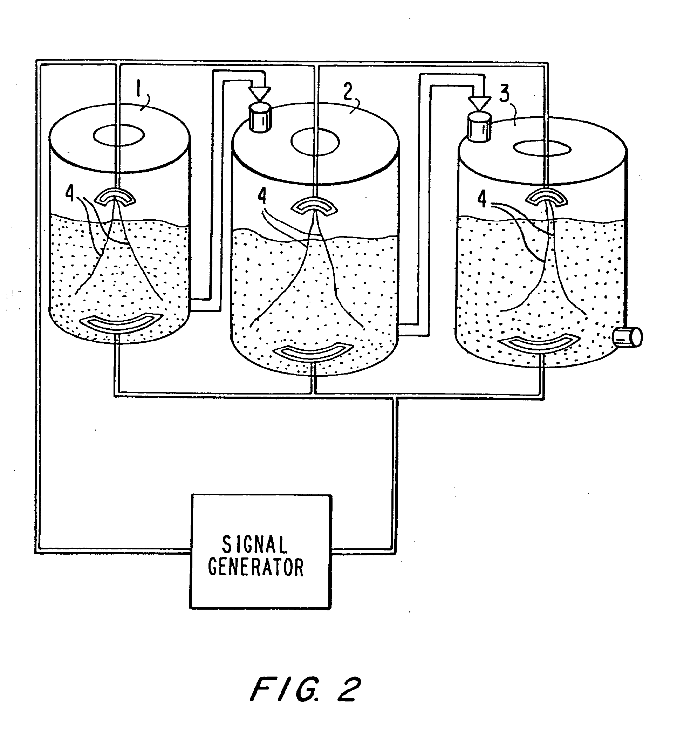

To prepare the yeast compositions of the invention, an apparatus depicted in Fig. 2 or an equivalent thereof can be used. This apparatus includes three containers, a first container (1), a second container (2), and a third container (3), each equipped with a pair of electrodes (4). One of the electrodes is a metal plate placed on the bottom of the containers, and the other electrode comprises a plurality of electrode wires evenly distributed in the space within the container to achieve even distribution of the electric field energy. All three pairs of electrodes are connected to a common signal generator.

The culture medium used for this purpose is a mixed fruit extract solution containing the following ingredients per 1000 L: 300 L of wild Chinese hawthorn extract, 300 L of jujube extract, 300 L of *Schisandra chinensis (Turez) Baill* seeds extract, and 100 L of soy bean extract. To prepare hawthorn, jujube and *Schisandra chinensis (Turez) Baill* seeds extracts, the fresh fruits are washed and dried under sterile conditions to reduce the water content to no higher than 8%. One hundred kilograms of the dried fruits are then ground (≥20 mesh) and added to 400 L of sterile water. The mixtures are stirred under sterile conditions at room temperature for twelve hours, and then centrifuged at 1000 rpm to remove insoluble residues. To make the soy bean extract, fresh soy beans are washed and dried under sterile conditions to reduce the water content to no higher than 8%. Thirty kilograms of dried soy beans are then ground into particles of no smaller than 20 mesh, and added to 130 L of sterile water. The mixture is stirred under sterile conditions at room temperature for twelve hours and centrifuged at 1000 rpm to remove insoluble residues. Once the mixed fruit extract solution is prepared, it is autoclaved at 121°C for 30 minutes and cooled to below 40°C before use.

One thousand grams of the activated yeast powder prepared as described above (Section V, *supra)* is added to 1000 L of the mixed fruit extract solution, and the yeast solution is transferred to the first container (1) shown in Fig. 2. The yeast cells are then cultured in the presence of an alternating electric field having a frequency of 11528 MHz and a field strength of about 420-450 mV/cm (eg., 435 mV/cm) at 28-32°C under sterile conditions for 12 hours. The yeast cells are further incubated in an alternating electric field having a frequency of 11535 MHz and a field strength of 420-450 mV/cm (e.g., 433 mV/cm). The culturing continues for another 10 hours.

The yeast culture is then transferred from the first container (1) to the second container (2) (if need be, a new batch of yeast culture can be started in the now available the first container (1)), and subjected to an alternating electric field having a frequency of 11528 MHz and a field strength of 320-350 mV/cm (e.g., 332 mV/cm) for 14 hours. Subsequently the frequency and field strength of the electric field are changed to 11535 MHz and 310-350 mV/cm (e.g., 310 mV/cm), respectively. The culturing process continues for another 12 hours.

The yeast culture is then transferred from the second container (2) to the third container (3), and subjected to an alternating electric field having a frequency of 11528 MHz and a field strength of 210-250 mV/cm (e.g:, 223 mV/cm) for 18 hours. Subsequently the frequency and field strength of the electric field are changed to 11535 MHz and 190-210 mV/cm (e.g., 200 mV/cm), respectively. The culturing continues for another 12 hours.

The yeast culture from the third container (3) can then be packaged into vacuum sealed bottles for use as dietary supplement. The compositions may conveniently be formulated as health drinks. If desired, the final yeast culture can also be dried within 24 hours and stored in powder form. The dietary supplement can be taken three to four times daily at 30~60 ml per dose for a three-month period, preferably 10-30 minutes before meals and at bedtime.

In some embodiments, the compositions of the invention can also be administered intravenously or peritoneally in the form of a sterile injectable preparation. Such a sterile preparation can be prepared as follows. A sterilized health drink composition is first treated under ultrasound (1000 Hz) for 10 minutes and then centrifuged at 4355 g for another 10 minutes. The resulting supernatant is adjusted to pH 7.2-7.4 using 1 M NaOH and subsequently filtered through a membrane (0.22 µm for intravenous injection and 0.45 µm for peritoneal injection) under sterile conditions. The resulting sterile preparation is submerged in a 35-38 °C water bath for 30 minutes before use.

The yeast compositions of the present invention are derived from yeasts used in food and pharmaceutical industries. The yeast compositions are thus devoid of side effects associated with many pharmaceutical compounds.

### VII. Examples

In order that this invention be more fully understood, the following examples are set forth. These examples are for the purpose of illustration only and are not to be construed as limiting the scope of the invention in any way.

The activated yeast compositions used in the following examples were prepared as described above, using *Saccharomyces cerevisiae* Hansen AS2.561, cultured in the presence of an alternating electric field having the electric field frequency and field strength exemplified in the parentheses following the recommended ranges listed in Section IV, *supra.* Control (i.e., untreated) yeast compositions were those prepared in the same manner as described in Section VI, *supra,* except that the yeast cells were cultured in the absence of EMFs. Unless otherwise specified, all compositions of interest were administered to the animals by intragastric feeding.

### Example 1: Control of Essential Hypsertension in Wistar Rat

To test the ability of the activated yeast compositions to reduce blood pressure, thirty-two healthy Wistar rats of average weight of about 200±20 g (8-10 months old, half of them male and the other half female) were randomly divided into four equal groups, Groups A, B, C and D. Blood pressure of each rat was measured with a manometer for three consecutive days. After three days, each rat was subcutaneously injected with 4 mg of testosterone propionate in 0.4 ml of sesame oil per day for fourteen consecutive days. At this point, the elevation in their blood pressure level was observed to be more than 1.3 kPa (10 mmHg).

A composition of interest (2 ml/kg/day) was then administered to each rat for sixteen consecutive days, while their blood pressure level was monitored every four days. Rats in Groups A, B, C and D received distilled water, reserpine, the activated yeast composition and the control yeast composition, respectively. The results were summarized in Table 2.

**Table 2**

| Group | Blood Pressure (kPa) | | | |
|---|---|---|---|---|
| | 1 | 2 | 3 | 4 |
| | Before Injection with Testosteronyl 17-propionate | After Injection with Testosteronyl 17-propionate | After Administration of a Composition | Change in Blood Pressure (Value in Col. 3 - Value in Col. 2) |
| A | 14.5±0.41 | 17.4±0.82 | 17.8±0.56 , | -0.4±0.26 |
| B | 14.7±0.32 | 16.7±0.56 | 15.3±0.89 | 1.4±0.33 |
| C | 14.3±0.23 | 16.5±0.52 | 12.3±0.68 | 4.2±0.16 |
| D | 14.6±0.27 | 16.4±0.54 | 17.5±0.51 | -1.1±0.03 |

The results in Table 2 show that the activated yeast composition was more effective in reducing blood pressure than both the known anti-hypertensive agent reserpine and the control yeast composition.

### Example 2: Control of Renal Hypertension in Wistar Rats

To test the ability of the activated yeast compositions to treat renal hypertension, thirty healthy male Wistar rats of average weight of about 250 to 300 g (12-14 months old) were randomly divided into three equal groups, Groups A, B and C. Under ether anesthesia, each rat was laid prone on an operating table and its posterior abdominal cavity was opened. The left kidney was separated from muscle tissues and the renal pedicle was clamped for the duration of the procedure (about four to five hours). Anesthesia pentobarbital (50 mg/kg) was injected into the abdominal cavity. The anesthetized rat was then turned over and its trachea was located and intubated. Heparin solution (0.1% heparin in saline, 0.15 ml/100g body weight) was injected into the carotid artery through a small catheter, which was subsequently sealed. A sphygmomanometer was connected to the carotid artery. The femoral vein was located and separated from the surrounding tissue and inserted with a small catheter equipped with a syringe containing a composition of interest (0.15 ml/100g body weight). The composition was then injected into the femoral vein within 10 seconds. Rats in Group A, B and C were injected with saline, the activated yeast composition and the control yeast composition, respectively. After the blood pressure readings stabilized, the clamp at the renal pedicle was released. The blood pressure readings were observed to gradually increase and the stabilized readings were recorded. The effects of the tested compositions on renal hypertension were summarized in Table 3.

**Table 3.**

| Blood Pressure Readings of Male Wistar Rats | | | | |
|---|---|---|---|---|
| Group | Before Administering a Composition | | After Administering a Composition | Monitoring Duration (minutes) |
| | Before Release of the Clamp | After Release of the Clamp | | |
| A | 15.2±0.8 | 19.2±1.7 | 19.8±1.3 | >50 |
| B | 15.6±0.9 | 19.6±1.3 | 11.5±1.4 | >50 |
| C | 15.4±0.7 | 19.8±1.2 | 18.7±1.7 | >50 |

The results in Table 3 show that unlike the control yeast composition, the activated yeast composition was effective in treating renal hypertension, namely, lowering blood pressure.

While a number of embodiments of this invention have been set forth, it is apparent that the basic constructions may be altered to provide other embodiments which utilize the compositions and methods of this invention.

## Claims

1. A composition comprising a plurality of yeast cells, wherein said plurality of yeast cells are **characterized by** their ability to treat hypertension in a subject, said ability resulting from their having been cultured in the presence of an alternating electric field having a frequency in the range of 11000 to 12000 to MHz and a field strength in the range of 180 to 500 mV/cm, as compared to yeast cells not having been so cultured.

2. The composition of claim 1, wherein said frequency is in the range of 11508 to 11535 MHz.

3. The composition of claim 1, wherein said field strength is in the range of 190-210, 210-250, 280-320, 320-350, 350-380, 380-420, or 420-450 mV/cm.

4. The composition of any one of claims 1 to 3, wherein said yeast cells are cells of the species *Saccharomyces cerevisiae, Saccharomyces carlsbergensis, Saccharomyces rouxii, Saccharomyces sake, Saccharomyces uvarum, Saccharomyces sp., Schizosaccharomyces pombe,* or *Rhodotorula aurantiaca.*

5. The composition of any preceding claim wherein said yeast cells are cells of the strain deposited at the China General Microbiological Culture Collection Center with an accession number selected from the group consisting of AS2.420, AS2.440, AS2.444, AS2.375, AS2.501, AS2.502, AS2.503, AS2.504, AS2.535, AS2.558, AS2.560, AS2.561, AS2.562, and IFFI1048.

6. The composition of any preceding claim wherein said composition is in the form of a tablet, powder, or a health drink.

7. The composition of claim 6, wherein said composition is in the form of a health drink.

8. The composition of any preceding claim wherein said hypertension is essential or secondary.

9. A method of preparing a yeast composition, comprising culturing a plurality of yeast cells in the presence of an alternating electric field having a frequency in the range of 11000 to 12000 MHz and a field strength in the range of 180 to 500 mV/cm for a period of time, wherein said composition is capable of treating hypertension in a subject.

10. The use of a composition according to any one of claims 1 to 8 in the manufacture of a medicament for the treatment of hypertension.
